# DEMANDE DE BREVET EUROPEEN

(11) **EP 1 543 816 A1**
(43) Date de publication de la demande: **22.06.2005**
(21) Numéro de dépôt: 04292663.4
(22) Date de dépôt: 10.11.2004
(51) Int. Cl.: A61K 7/075

(54) **Composition détergente contenant au moins un polymère semi cristallin, un tensio-actif anionique et/ou non ionique et au moins une huile**

(30) Priorité: 19.12.2003 FR 0351142
(71) Demandeur: L'OREAL, 75008 Paris (FR)
(72) Inventeur: Parris, Eric, 92110 Clichy (FR); Gawtrey, Jonathan, 92100 Boulogne (FR)
(74) Mandataire: Le Blainvaux, Françoise

(57) **Abrégé**

La présente invention décrit une composition détergente comprenant, dans un milieu cosmétiquement acceptable du polymère semi-cristallin associé à au moins une huile, et au moins un tensioactif détergent anionique et/ou non ioniques.

Cette composition peut être utilisée comme shampoing et permet d'obtenir de bonnes propriétés de conditionnement des cheveux.

## Description

La présente invention concerne une composition détergente contenant au moins un polymère semi-cristallin associé à au moins une huile, et un procédé de traitement cosmétique notamment capillaire mettant en oeuvre cette composition.

Dans le domaine de la cosmétique, on cherche notamment à améliorer le conditionnement des cheveux. Par conditionnement, on entend des propriétés de démêlage facile, de brillance, de douceur au toucher, de lissage (toucher et visuel) et de glissant.

L'utilisation de polymère semi-cristallin est connue en cosmétique, et plus particulièrement dans le domaine du maquillage comme cela est décrit dans les demandes de brevet FR2824267. Des compositions cosmétiques dont la phase grasse est gélifiée par des polymères semi-cristallins ont été décrites la demande FR 2824264 qui décrit plus particulièrement des compositions solides de rouge à lèvre sous la forme d'un stick .

Les brevets US 5 736 125 et US 5 156 911, ainsi que la demande WO 01/19333 illustrent certains types de polymères semi-cristallins qui peuvent entrer dans la composition des formules de l'invention. Néanmoins, ces documents ne décrivent pas de compositions cosmétiques détergentes.

La Demanderesse a trouvé de manière surprenante qu'en utilisant, dans des compositions détergentes de traitement capillaire, au moins un polymère semi-cristallin en association avec au moins une huile, il était possible d'obtenir un très bon effet de conditionnement des cheveux.

Les cheveux traités avec cette composition sont lisses, se démêlent facilement sont brillants, souples, individualisés et ont un toucher doux et sans résidus.

Sans être tenu par une quelconque théorie, il semblerait que dans ces conditions, on augmente significativement le dépôt d'huile sur les cheveux, d'où une efficacité accrue. Cette amélioration se fait cependant sans avoir un toucher chargé gras, ce qui est le cas habituellement lorsqu'on augmente la quantité d'huile.

Par ailleurs, cet effet de conditionnement peut être rémanent au rinçage .

L'invention a donc pour objet une composition détergente notamment cosmétique comprenant, dans un milieu cosmétiquement acceptable, au moins un tensioactif détergent anionique ou non ionique, au moins un polymère semi-cristallin ayant un point de fusion supérieur ou égal à 30 °C associé à au moins une huile.

Un objet supplémentaire de la présente invention est un procédé de traitement cosmétique des matières kératiniques mettant en oeuvre une composition selon l'invention.

D'autres objets, caractéristiques, aspects et avantages de l'invention apparaîtront encore plus clairement à la lecture de la description et des divers exemples qui suivent.

Selon l'invention, la composition détergente comprend au moins un polymère semi-cristallin associé à au moins une huile, et au moins un tensioactif détergent anionique ou non ionique.

Selon un mode particulier de l'invention, l'huile est, de préférence, pré-épaissie par le polymère semi-cristallin, c'est à dire qu'on mélange l'huile et le polymère semi-cristallin avant l'introduction dans la composition.

Le rapport pondéral huile(s)/polymère semi-cristallin(s) est de préférence supérieur ou égal à 50/50, mieux encore supérieur ou égal à 60/40, plus préférentiellement compris entre 60/40 et 99/1 et encore plus préférentiellement compris entre 80/20 et 99/1.

Cette huile, de préférence pré-épaissie est dispersée sous forme de particules dans la composition aqueuse. Les particules d'huile présentent de préférence une taille primaire moyenne en nombre allant de 1 à 100 µm (microns), mieux encore de 5 à 30 µm, et encore plus particulièrement de 10 à 20 µm (microns).

Au sens de la présente invention, on entend par "taille primaire de particule", la dimension maximale qu'il est possible de mesurer entre deux points diamétralement opposés d'une particule individuelle. La taille peut être déterminée, par exemple, par microscopie électronique à transmission ou à partir de la mesure de la surface spécifique par la méthode BET ou bien par l'intermédiaire d'un granulomètre laser.

Par "polymère semi-cristallin", on entend au sens de l'invention, des polymères comportant une partie cristallisable et une partie amorphe dans le squelette et présentant une température de changement de phase réversible du premier ordre, en particulier de fusion (transition solide-liquide). La partie cristallisable est soit une chaîne latérale (ou chaîne pendante), soit une séquence dans le squelette.

Par "polymères", on entend au sens de l'invention des composés comportant au moins 2 motifs de répétition, de préférence au moins 3 motifs de répétition et plus spécialement au moins 10 motifs répétitifs.

Lorsque la partie cristallisable est une séquence du squelette polymérique, cette séquence cristallisable est de nature chimique différente de celle des séquences amorphes; le polymère semi-cristallin est dans ce cas un copolymère séquence par exemple du type dibloc, tribloc ou multibloc. Lorsque la partie cristallisable est une chaîne pendante au squelette, le polymère semi cristallin peut être un homopolymère ou un copolymère.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère.

Par "composé organique" ou "à structure organique", on entend des composés contenant des atomes de carbone et des atomes d'hydrogène et éventuellement des hétéroatomes comme S, O, N, P seuls ou en association.

### Les polymères semi-cristallins

De façon avantageuse, le ou les polymères semi-cristallins de la composition de l'invention ont une masse moléculaire moyenne en poids Mp supérieure ou égale à 1000, allant notamment de 5 000 à 1 000 000, de préférence de 10 000 à 500 000, préférentiellement de 15 000 à 500 000.

De préférence, le polymère semi-cristallin comporte i) un squelette polymérique et ii) au moins une chaîne latérale cristallisable et/ou une séquence organique cristallisable faisant partie du squelette dudit polymère semi-cristallin.

En particulier, le polymère semi-cristallin est choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

Le ou les polymères semi-cristallins selon l'invention servant d'agent structurant sont des solides à température ambiante (25°C) et pression atmosphérique (760 mm de Hg), dont la température de fusion est supérieure ou égale à 30°C, de préférence allant de 30 °C à 80 °C), et plus préférentiellement allant de 30 °C à 70 °C. Cette température de fusion est une température de changement d'état du premier ordre. Cette température de fusion peut être mesurée par toute méthode connue et en particulier à l'aide d'un calorimètre à balayage différentiel (D.S.C).

Les valeurs de point de fusion correspondent notamment au point de fusion mesuré à l'aide d'un calorimètre à balayage différentiel (D. S. C), tel que le calorimètre vendu sous la dénomination DSC 30 par la société METTLER, avec une montée en température de 5 ou 10°C par minute. (Le point de fusion considéré est le point correspondant à la température du pic le plus endotherme du thermogramme).

Le ou les polymères semi-cristallins selon l'invention ont de préférence une température de fusion supérieure à la température du support kératinique destiné à recevoir ladite composition, en particulier les cheveux.

Le ou les polymères semi-cristallins selon l'invention sont capables de structurer (d'épaissir) seuls ou en mélange, l'huile sans ajout de tensioactif particulier, ni de sel.

Selon l'invention les polymères semi-cristallins sont avantageusement solubles dans la phase grasse, notamment à au moins 1 % en poids, à une température supérieure à leur température de fusion. En dehors des chaînes ou séquences cristallisables, les séquences des polymères sont amorphes.

De préférence, le squelette polymérique des polymères semi-cristallins est soluble dans la phase grasse liquide.

De préférence, les séquences ou chaînes cristallisables des polymères semi-cristallins représentent au moins 30 % du poids total de chaque polymère et mieux au moins 40 %. Les polymères semi-cristallins à chaînes latérales cristallisables sont des homo ou des copolymères. Les polymères semi-cristallins de l'invention à séquences cristallisables sont des copolymères, séquencés ou multiséquencés. Ils peuvent être obtenus par polymérisation de monomère(s) à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins de l'invention sont d'origine synthétique. En outre, de préférence, ils ne comportent pas de squelette polysaccharidique.

Les polymères semi-cristallins utilisables dans l'invention sont en particulier :
- les copolymères séquencés de polyoléfines à cristallisation contrôlée, dont les monomères sont décrits dans EP-A-0 951 897.
- les polycondensats et notamment de type polyester, aliphatique ou aromatique ou les copolyesters aliphatique/aromatique,
- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré(s), tels que décrits dans le document WO-A-01/19333,
- et leurs mélanges. Dans ces deux derniers cas, la ou les chaînes latérales ou séquences cristallisables sont hydrophobes.

### A) Polymères semi-cristallins à chaînes latérales cristallisables

On peut citer en particulier ceux définis dans les documents US-A-5 156 911 et WO-A-01/19333.
. Ce sont des homopolymères ou copolymères comportant de 50 à 100 % en poids de motifs résultant de la polymérisation d'un ou plusieurs monomères porteurs de chaîne latérale hydrophobe cristallisable.
. Ces homo- ou co-polymères sont de toute nature du moment qu'ils présentent les conditions indiquées ci-après avec en particulier la caractéristique d'être solubles ou dispersables dans la phase grasse liquide, par chauffage au-dessus de leur température de fusion Pf. Ils peuvent résulter :
   - de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
   - de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.
      a) D'une façon générale les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins. Ces polymères sont choisis notamment parmi les homopolymères et copolymères résultant de la polymérisation d'au moins un monomère à chaîne(s) cristallisable(s) qui peut être représenté par la formule (I) : 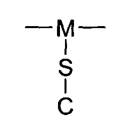
   avec M représentant un atome du squelette polymérique
   S représentant un espaceur
   C représentant un groupe cristallisable

Les chaînes « -S-C » cristallisables peuvent être aliphatiques ou aromatiques, éventuellement fluorées ou perfluorées. « S » représente notamment un groupe (CH₂)ₙ ou (CH₂CH₂O)ₙ ou (CH₂O)ₙ, linéaire ou ramifié ou cyclique, avec n entier allant de 0 à 22. De préférence « S » est un groupe linéaire. De préférence, « S » et « C » sont différents.

Lorsque les chaînes cristallisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyle en C₁₄-C₂₄. de préférence en C₁₆-C₂₂ .Lorsqu'il s'agit de chaînes alkyle fluorées ou perfluorées, elles comportent au moins 11 atomes de carbone dont au moins 6 atomes de carbone sont fluorés.

Comme exemple d'homopolymères ou de copolymères semi-cristallins à chaîne(s) cristallisable(s), on peut citer ceux résultant de la polymérisation d'un ou plusieurs monomères suivants : les (méth)acrylates d'alkyle saturés avec le groupe alkyle en C₁₄-C₂₄, les (méth)acrylates de perfluoroalkyle avec un groupe alkyle perfluoro en C₁₁-C₁₅, les N-alkyl (méth)acrylamides avec le groupe alkyle en C₁₄ à C₂₄ avec ou sans atome(s) de fluor, les esters vinyliques à chaînes alkyle ou perfluoro (alkyle) avec le groupe alkyle en C₁₄ à C₂₄ (avec au moins 6 atomes de fluor pour une chaîne perfluoro alkyle), les éthers vinyliques à chaînes alkyle ou perfluoro (alkyle) avec le groupe alkyle en C₁₄ à C₂₄ et au moins 6 atomes de fluor pour une chaîne perfluoro alkyle, les alpha-oléfines en C₁₄ à C₂₄ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50 % de groupes Y ou Z résultant de la copolymérisation :
α ) de Y qui est un monomère polaire ou non polaire ou un mélange des deux :
   . Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth)acrylamide, un N-alkyl(méth)acrylamide, un N,N-dialkyl(méth)acrylamide comme par exemple le N,N-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.
   . Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou le styrène substitué par un groupe alkyle en C₁ à C₁₀, comme l'α-méthylstyrène, un macromonomère du type polyorganosiloxane à insaturation vinylique.

   Par "alkyle", on entend au sens au sens de l'invention un groupement saturé notamment en C₈ à C₂₄, sauf mention exprès.
β) de Z qui est un monomère polaire ou un mélange de monomères polaires.
   Dans ce cas, Z a la même définition que le "Y polaire" défini ci-dessus.

De préférence, les polymères semi-cristallins à chaîne latérale cristallisable sont des homopolymères d'alkyl(méth)acrylate ou d'alkyl(méth)acrylamide avec un groupe alkyle tel que défini ci-dessus, et notamment en C₁₄-C₂₄, des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

### B) Les polymères portant dans le squelette au moins une séquence cristallisable

Il s'agit encore de polymères solubles ou dispersables dans la phase grasse liquide par chauffage au-dessus de leur point de fusion Pf. Ces polymères sont notamment des copolymères séquencés constitués d'au moins deux séquences de nature chimique différente dont l'une est cristallisable.
- On peut utiliser les polymères définis dans le brevet US-A-5 156 911,
- Les copolymères séquencés d'oléfine ou de cyclooléfine à chaîne cristallisable comme ceux issus de la polymérisation séquencée de :
   . cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges,
   . avec l'éthylène, le propylène, le 1-butène, le 3-méthyl-1-butène, le 1-hexène, le 4-méthyl-1-pentène, le 1-octène, le 1-décène, le 1-éicosène ou leurs mélanges,
   . et en particulier les copoly(éthylène/norbornène) blocs et les terpolymères (éthylène/propylène/éthylidène-norbornène), blocs. On peut aussi utiliser ceux résultants de la copolymérisation séquencée d'au moins 2 α-oléfines en C₂-C₁₆ et mieux en C₂-C₁₂ tels que ceux cités précédemment et en particulier les bipolymères séquencés d'éthylène et d'1-octène.
- Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
   . Séquence cristallisable par nature : a) polyester comme les poly(alkylène téréphtalate), b) polyoléfine comme les polyéthylènes ou polypropylènes.
   . Séquence amorphe et lipophile comme : les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe, on peut citer :
α) les copolymères séquencés poly(ε-caprolactone)-b-poly(butadiène), utilisés de préférence hydrogénés, tels que ceux décrits dans l'article "Melting behavior of poly(ε-caprolactone)-block-polybutadiène copolymers" de S. Nojima, Macromolécules, 32, 3727-3734 (1999).
β) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).
γ) les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993) et "Polymer agregates with crystalline cores : the system poly(ethylene)-poly(ethylene-propylene)" P. Richter et al., Macromolécules, 30, 1053-1068 (1997).
δ) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins de la composition de l'invention peuvent être ou non réticulés en partie du moment où le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.

De préférence, les polymères semi-cristallins utilisables selon l'invention ne sont pas réticulés.

Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en C₁₄ à C₂₄, les (méth)acrylates de perfluoroalkyle en C₁₁ à C₁₅, les N alkyl (méth)acrylamides en C₁₄ à C₂₄ avec ou sans atome(s) de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alphaoléfines en C₁₄ à C₂₄, les para-alkyl styrènes avec un groupe alkyle comportant de 12 à 24 atomes de carbone, avec au moins un ester ou amide d'acide monocarboxylique en C₁ à C₁₀ éventuellement fluoré, qui peut être représenté car la formule suivante : 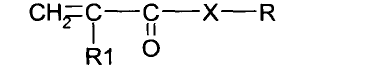 dans laquelle R₁ est H ou CH₃, R représente un groupe alkyle en C1-C10 éventuellement fluoré et X représente O, NH ou NR₂, où R₂ représente un groupe alkyle en C₁-C₁₀ éventuellement fluoré.

A titre d'exemple particulier de polymère semi-cristallins structurant utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C). Ils sont porteurs de chaînes latérales cristallisables et présentent la formule (I) précédente.

Dans la suite de la description, le ou les polymères semi-cristallins ayant une température de fusion Pf₂ inférieure à 50°C seront dénommés "polymères à bas point de fusion" et le ou les composés cristallins ou semi-cristallins ayant une température de fusion Pf₁ supérieure ou égale à 50°C seront dénommés "composés à haut point de fusion". Selon l'invention, le point de fusion peut être mesuré notamment par toute méthode connue et en particulier avec un calorimètre à balayage différentiel (D.S.C).

Selon l'invention le ou les composés semi-cristallins à haut point de fusion sont avantageusement des polymères ayant une température de fusion Pf₁ telle que 50°C ≤ Pf₁ ≤ 150°C, mieux 55°C ≤ Pf₁ ≤ 150°C, et de préférence 60°C ≤ Pf₁ ≤ 130°C et les polymères à bas point de fusion ont avantageusement une température de fusion Pf₂ telle que 30°C ≤ Pf₂ < 50°C, et mieux 35°C ≤ Pf₂ ≤ 45°C. Cette température de fusion est une température de changement d'état du premier ordre.

De façon générale, les polymères à bas point de fusion présentent une température de fusion Pf₂ au moins égale à la température du support kératinique devant recevoir la composition selon l'invention.

Comme polymère semi-cristallin à haut point de fusion utilisable dans l'invention, on peut citer des polymères cristallins, solides à température ambiante et ayant une température de fusion supérieure à 50°C comme des polymères statistiques comportant une cristallisation contrôlée, tels que décrits dans le document EP-A-0 951 897 et plus spécialement les produits commerciaux Engage 8 401 et Engage 8 402 de Dupont de Nemours, respectivement de température de fusion de 51°C et 64°C et qui sont des bipolymères statistiques éthylène/1-octène.
i) Les polymères semi-cristallins présentant un point de fusion supérieur ou égal à 50°C sont notamment l'Intelimer décrit dans la brochure "Intelimer® polymers", Landec IP22 de température de fusion de 56°C, qui est un produit visqueux à température ambiante.
   On peut aussi utiliser les polymères semi-cristallins obtenus par copolymérisation de l'acrylate de béhényle et de l'acide acrylique ou de NVP, tels que décrits dans les documents US-A-5 519 063 et EP-A- 055 0745 et plus spécialement ceux décrits dans les exemples 3 et 4, ci-après, de préparation de polymère.
   ii) Les polymères semi-cristallins dont le point de fusion est inférieur à 50°C sont notamment ceux décrits dans les exemples 3, 4, 5, 7, 9 du brevet US-A-5 156 911 à groupement -COOH, résultant de la copolymérisation d'acide acrylique et d'alkyl(méth)acrylate en C₅ à C₁₆ (de température de fusion allant de 20°C à 35°C) et plus particulièrement résultant de la copolymérisation :
   . d'acide acrylique, d'hexadécylacrylate et d'isodécylacrylate dans un rapport 1/16/3,
   . d'acide acrylique et de pentadécylacrylate dans un rapport 1/19,
   . d'acide acrylique, d'hexadécylacrylate, éthylacrylate dans un rapport 2,5/76,5/20,
   . d'acide acrylique, d'hexadécylacrylate et de méthylacrylate dans un rapport 5/85/10,
   . d'acide acrylique, d'octadécylméthacrylate dans un rapport 2,5/97,5,
d'hexadécylacrylate, de monométhyl éther de méthacrylate polyéthylèneglycol à 8 motifs d'éthylèneglycol, et d'acide acrylique dans un rapport pondéral 8,5/1/0,5.

On peut aussi utiliser le polymère Structure « O » de National Starch tel que celui décrit dans le document US-A-5 736 125 de température de fusion de 44°C ainsi que les polymères semi-cristallins à chaînes pendantes cristallisables comportant des groupements fluorés tels que décrits dans les exemples 1, 4, 6, 7 et 8 du document WO-A-01/19333.

On peut encore utiliser les polymères semi-cristallins de bas point de fusion obtenus par copolymérisation d'acrylate de stéaryle et d'acide acrylique ou de NVP tels que décrits dans le document US-A-5519063 ou EP-A-550745 et plus spécialement ceux décrits dans les exemples 1 et 2, ci-après, de préparation de polymère, de température de fusion respectivement de 40°C et 38°C.

On peut aussi utiliser les polymères semi-cristallins obtenus par copolymérisation de l'acrylate de béhényle et de l'acide acrylique ou de NVP tels que décrits dans les documents US-A-5519063 et EP-A-550745.

De préférence, les polymères semi-cristallins à bas point de fusion et/ou ceux à haut point de fusion ne comportent pas de groupement carboxylique.

Selon un mode plus particulier de réalisation de l'invention, le polymère est issu d'un monomère à chaîne cristallisable choisi parmi les (méth)acrylates d'alkyle saturés en C₁₄ à C₂₄ et encore plus particulièrement les poly(acrylate de stéaryle) ou les poly(acrylate de béhényle).

L'épaississement de la phase grasse est modulable selon la nature du ou des polymères et leurs concentrations et peut être telle que l'on obtienne de préférence une viscosité allant de 1000 à 250.000 cps et de préférence de 10.000 à 50.000 cps. Mesurée à 25°C avec un appareil RHEOMAT 180 avec un taux de cisaillement de 100s⁻¹ .

Le polymère semi-cristallin tel que défini ci-dessus est de préférence présent en une quantité comprise entre 0,005 et 20 % en poids, mieux encore en une quantité comprise entre 0,05 et 10 % en poids, et encore plus préférentiellement en une quantité comprise entre 0,1 et 5 % en poids par rapport au poids total de la composition.

Par "huile", au sens de la demande, on entend un corps gras insoluble dans l'eau, liquide à température ambiante (25°C) et pression atmosphérique (760 mm de Hg. La phase huileuse peut être composée d'une ou plusieurs huiles compatibles entre elles.

Par insoluble dans l'eau, on entend au sens de la présente invention, une substance qui présente une solubilité dan l'eau pure inférieure à 1 % à 25 °C et sous pression atmosphérique.

Les huiles utilisées dans la présente invention présente une viscosité dynamique à 25 °C, inférieure à 1 Pa.s (1000 cps), de préférence comprise entre 10⁻³ et 0,1 Pa.s (1 et 100 cps). La viscosité dynamique est mesurée à 25 °C avec un taux de cisaillement de 100 s⁻¹, par exemple, avec l'appareil référencé RM 180 Rheomat de la société METTLER.

Les huiles pouvant être utilisées dans la présente invention sont choisies de préférence, parmi les huiles végétales, les huiles minérales, les huiles de synthèse, et les esters d'acide gras.

Parmi les huiles végétales pouvant être utilisées dans la présente invention, on peut notamment citer l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot et l'huile de calophyllum.

Des exemples d'huiles minérales sont l'huile de paraffine et l'huile de vaseline.

Les huiles de synthèse peuvent notamment être choisies parmi les polydécènes, le squalane, les poly(alpha-oléfines) comme l'isododécane ou l'isohexadécane, les huiles végétales transestérifiées et les huiles fluorées.

On peut également utiliser des esters d'acide gras, tels que par exemple, les composés de formule RₐCOOR_{b} dans laquelle Rₐ représente le reste d'un acide gras supérieur comportant de 5 à 29 atomes de carbone et R_{b} représente une chaîne hydrocarbonée contenant de 3 à 30 atomes de carbone, telles que l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyldodécyle.

Les huiles particulièrement préférées dans le cadre de la présente invention sont l'huile d'avocat, l'huile de ricin, l'huile d'olive, le polydécène hydrogéné, le myristate d'isopropyle, l'isononanoate d'isononyle, une paraffine liquide

La ou les huile(s) telle(s) que définie(s) ci-dessus est ou sont de préférence présente(s) en une quantité comprise entre 0,01 et 30 % en poids, encore plus préférentiellement en une quantité comprise entre 0,1 et 15 % en poids par rapport au poids de la composition, et plus particulièrement de 0,5 à 10% en poids.

Les tensioactifs détergents anioniques ou non ioniques ont de préférence une HLB supérieure ou égale à 8, de préférence supérieure à 12 et de préférence inférieure à 30.

A titre de tensioactifs détergents anioniques utilisables, seuls ou en mélanges, dans le cadre de la présente invention, on peut citer notamment les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les sulfates d'alkyle, les alkyléther-sulfates, les alkylamidoéthersulfates, les alkylarylpolyéthersulfates, les monoglycéride-sulfates ; les alkylsulfonates, les phosphates d'alkyle, les alkylamidesulfonates, les alkylarylsulfonates, les alpha-oléfine-sulfonates, les paraffine-sulfonates ; les sulfosuccinates d'alkyle, les alkyléthersulfosuccinates, les alkylamidesulfosuccinates ; les sulfoacétates d'alkyle ; les acylsarcosinates ; et les acylglutamates, les groupes alkyle ou acyle de tous ces composés comportant de 6 à 24 atomes de carbone et le groupe aryle désignant de préférence un groupe phényle ou benzyle. On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les sulfosuccinamates d'alkyle, les iséthionates d'acyle et les N-acyltaurates, le groupe alkyle ou acyle de tous ces composés comportant de 12 à 20 atomes de carbone. Parmi les tensioactifs anioniques encore utilisables, on peut également citer les lactylates d'acyle dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Parmi les tensioactifs anioniques cités ci-dessus, on préfère utiliser selon l'invention les sels, en particulier de sodium, de magnésium ou d'ammonium, de sulfates d'alkyle ; d'alkyléther-sulfates, comme le lauryléthersulfate de sodium, de préférence à 2 ou 3 moles d'oxyde d'éthylène ; d'alkyléthercarboxylates ; et leurs mélanges, les groupes alkyle comportant généralement de 6 à 24 atomes de carbone, et de préférence de 8 à 16 atomes de carbone.

Les agents tensioactifs non-ioniques utilisables dans la composition selon l'invention, sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides et plus particulièrement les alkyl(C₈-C₁₆)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)aminopropylmorpholine ; et leurs mélanges.

En ce qui concerne les alkypolyglycosides, ces composés sont bien connus et peuvent être plus particulièrement représenté par la formule générale suivante (A):

R₁O-(R₂O)ₜ (G)ᵥ

dans laquelle R₁ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, R₂ représente un radical alkylène comportant environ de 2 à 4 atomes de carbone, G représente un motif sucre comportant de 5 à 6 atomes de carbone, t désigne une valeur allant de 0 à 10, de préférence 0 à 4, de préférence 0 à 4 et v désigne une valeur allant de 1 à 15.

Des alkylpolyglycosides préférés selon la présente invention sont des composés de formule (A) dans laquelle R₁ désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation, i.e. la valeur de v dans la formule (A), peut aller de 1 à 15, de préférence de 1 à 4. Le degré moyen de polymérisation est plus particulièrement compris entre 1 et 2.

Les liaisons glycosidiques entre les motifs sucre sont de type 1-6 ou 1-4 et de préférence 1-4.

Des composés de formule (A) sont notamment représentés par les produits vendus par la société COGNIS sous les dénominations PLANTAREN® (600 CS/U, 1200 et 2000) ou PLANTACARE® (818, 1200 et 2000). On peut également utiliser les produits vendus par la société SEPPIC sous les dénominations TRITON CG 110 (ou ORAMIX CG 110) et TRITON CG 312 (ou ORAMIX® NS 10), les produits vendus par la société B.A.S.F. sous la dénomination LUTENSOL GD 70 ou encore ceux vendus par la société CHEM Y sous la dénomination AG10 LK.

On peut également utiliser par exemple, l'Alkyl en C8/C16 polyglucoside 1,4 en solution aqueuse à 53% commercialisé par COGNIS sous la référence PLANTACARE® 818 UP.

En ce qui concerne les tensioactifs mono ou polyglycérolés, ils comportent de préférence en moyenne de 1 à 30 groupements glycérol, plus particulièrement de 1 à 10 groupements glycérol et en particulier de 1,5 à 5.

Les tensioactifs monoglycérolés ou polyglycérolés sont de préférence choisis parmi les composés de formule suivantes : RO[CH₂CH(CH₂OH)O]ₘH , RO[CH₂CH(OH)CH₂O]ₘH ou RO[CH(CH₂OH)CH₂O]ₘH ; dans laquelle R représente un radical hydrocarboné saturé ou insaturé, linéaire ou ramifié, comportant de 8 à 40 atomes de carbone et de préférence de 10 à 30 atomes de carbone ; m est un nombre compris entre 1 et 30, de préférence entre 1 et 10, plus particulièrement de 1,5 à 6.

R peut éventuellement comprendre des hétéroatomes tels que par exemple oxygène et azote. En particulier, R peut éventuellement comprendre un ou plusieurs groupements hydroxy et/ou éther et/ou amide.

R désigne de préférence des radicaux alkyle et/ou alkényle en C10-C20, éventuellement mono ou polyhydroxylé.

On peut utiliser par exemple, l'hydroxylauryléther polyglycérolé (3.5 moles) commercialisé sous la dénomination Chimexane® NF de Chimex.

Parmi les tensioactifs non-ioniques cités ci-dessus, on utilise de préférence les alky)(C₆-C₂₄)polyglycosides, les alcools polyglycérolés, les alpha-diols polyglycérolés et leurs mélanges.

Le ou les tensioactifs anioniques et/ou non-ioniques sont généralement présents en une quantité comprise entre 3 et 50 % en poids, de préférence entre 4 et 30 % en poids, encore plus préférentiellement entre 5 et 20 % en poids par rapport au poids total de la composition détergente et encore plus particulièrement entre 10 et 18% en poids.

La composition détergente selon l'invention peut comprendre en outre un ou plusieurs tensioactifs zwittérioniques et/ou amphotères et éventuellement des tensioactifs non détergents en particulier non ioniques.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant, au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL®, tels que décrits dans les brevets US-2 528 378 et US-2 781 354 et classés dans le dictionnaire CTFA, 3^{ème} édition, 1982, sous les dénominations Amphocarboxy-glycinate et Amphocarboxypropionate de structures respectives (2) et (3) :

R₂-CONHCH₂CH₂-N⁺(R₃)(R₄)(CH₂COO⁻) (2)

dans laquelle :
R₂ représente un groupe alkyle dérivé d'un acide R₂-COOH présent dans l'huile de coprah hydrolysée, un groupe heptyle, nonyle ou undécyle,
R₃ représente un groupe bêta-hydroxyéthyle, et
R₄ représente un groupe carboxyméthyle ;
et

R₂-CONHCH₂CH₂-N(B)(C) (3)

dans laquelle :
B représente -CH₂CH₂OX',
C représente -(CH₂)₂-Y', avec z = 1 ou 2,
X' représente le groupe -CH₂CH₂-COOH ou un atome d'hydrogène,
Y' représente -COOH ou le groupe -CH₂-CHOH-SO₃H,
R_{2'} représente un groupe alkyle d'un acide R₂ -COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL® C2M concentré par la société RHODIA.

Parmi les tensioactifs amphotères, on utilise de préférence les alkyl(C₈-C₂₀)bétaïnes, les alkyl(C₈-C₂₀)amidoalkyl(C₆-C₈)bétaïnes, les alkylamphodiacétates et leurs mélanges.

Les tensioactifs amphotères et/ou non ioniques sont éventuellement présents en une quantité comprise entre 0,1 et 20 % en poids, de préférence entre 0,5 et 15 % en poids, encore plus préférentiellement entre 1 et 10 % en poids par rapport au poids total de la composition détergente.

La composition selon l'invention peut notamment comprendre en outre un ou plusieurs polymères cationiques. Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère comprenant des groupements cationiques et/ou des groupements ionisables en groupements cationiques.

Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 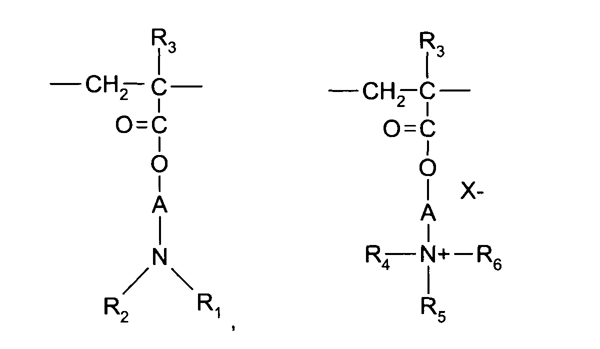 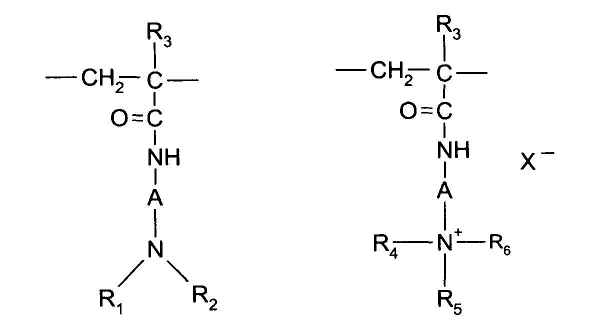 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyl-triméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyl-triméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymères vinylpyrrolidone / méthacrylamidopropyl dimethylamine commercialisés notamment sous la dénomination STYLEZE CC 10 par ISP.
   - et les copolymères vinylpyrrolidone / méthacrylamide de diméthylaminopropyle quaternisé tel que le produit vendu sous la dénomination "GAFQUAT HS 100" par la société ISP.
(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires sont par exemple décrits dans le brevet français 1 492 597, et sont en particulier les polymères commercialisés sous les dénominations "JR" (JR 400, JR 125, JR 30M) ou "LR" (LR 400, LR 30M) par la Société AMERCHOL. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
   Les gommes de galactomannane cationiques sont décrites plus particulièrement dans les brevets US 3 589 578 et 4 031 307 en particulier les gommes de guar comprenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société RHODIA.
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(7) les cyclopolymères d'alkyl diallyl amine ou de dialkyl diallyl ammonium tels que les homopolymères ou copolymères comportant comme constituant principal de la chaîne des motifs répondant aux formules (II) ou (III) : 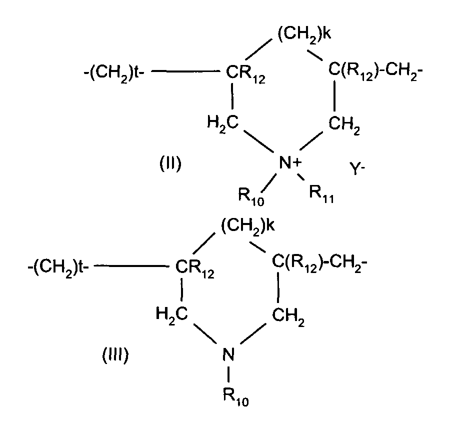 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "MERQUAT 100" par la société NALCO (et ses homologues de faibles masses molaires moyenne en poids) et les copolymères de chlorure de diallyldiméthylammonium et d'acrylamide commercialisés sous la dénomination "MERQUAT 550".
(8) les polymères de diammonium quaternaire comprenant des motifs récurrents répondant à la formule (IV): 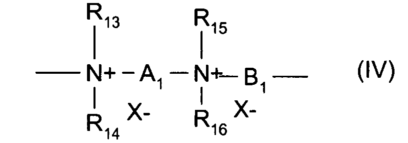 formule (IV) dans laquelle :
   R₁₃, R₁₄, R₁₅ et R₁₆, identiques ou différents, représentent des radicaux aliphatiques, alicycliques, ou arylaliphatiques comprenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles comprenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques comprenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ;

   De préférence, X⁻ est un anion tel que le chlorure ou le bromure.
   Ces polymères ont une masse molaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (V): 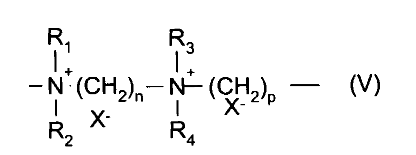 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
   Un composé de formule (V) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(9) les polymères de polyammonium quaternaires comprenant des motifs de formule (VI): 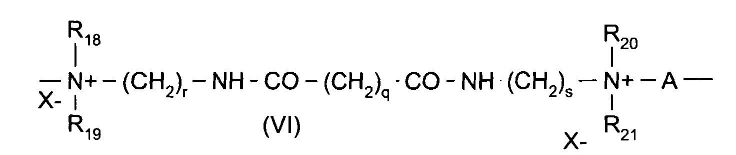 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogènure,
   A désigne un radical d'un dihalogénure ou représente de préférence -CH₂-CH₂-O-CH₂-CH₂-.
   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
(10) Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polyamines comme le Polyquart® H vendu par COGNIS, référencé sous le nom de « POLYETHYLENEGLYCOL (15) TALLOW POLYAMINE » dans le dictionnaire CTFA.
(12) Les polymères de préférence réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium comprenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, convenant dans l'invention, on peut utiliser de préférence les dérivés d'éther de cellulose quaternaires tels que les produits vendus sous la dénomination « JR 400 » par la Société AMERCHOL, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole, les homopolymères ou copolymères éventuellement réticulés de sels de méthacryloyloxyalkyl(C₁-C₄) trialkyl(C₁-C₄)ammonium et leurs mélanges.

Le ou les polymères cationiques sont généralement présents à des concentrations allant de 0,01 à 20%, de préférence de 0,05 à 10% et plus particulièrement de 0,1 à 5 % en poids par rapport au poids total de la composition.

Par milieu cosmétiquement acceptable, on entend un milieu compatible avec les cheveux, mais aussi d'odeur, d'aspect et de toucher agréables.

Le milieu cosmétiquement acceptable comprend l'eau ou un mélange d'eau et d'un solvant cosmétiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

De préférence, la composition comprend de 70 à 95 % en poids d'eau par rapport au poids total de la composition.

Les compositions de lavage selon l'invention présentent un pH final généralement compris entre 3 et 10. De préférence, ce pH est compris entre 4,5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants (associatifs ou non associatifs). On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères d'acide acrylique tels que les copolymères d'acide acrylique et d'acrylates d'alkyle en C₁₀-C₃₀ réticulés. Ces agents régulateurs de viscosité sont utilisés dans les composition selon l'invention dans des proportions pouvant aller jusqu'à 10 % en poids par rapport au poids total de la composition.

Les compositions conformes à l'invention peuvent également contenir de préférence jusqu'à 5 % d'agents nacrants ou opacifiants bien connus dans l'état de la technique tels que par exemple les alcools gras supérieurs à C16, les dérivés acylés à chaîne grasse tels que les monostéarates ou distéarates d'éthylène glycol ou de polyéthylèneglycol, les éthers à chaînes grasses (C10-C30) tels que par exemple le distéaryléther ou le 1-(hexadécyloxy)-2-octadécanol.

Les compositions conformes à l'invention peuvent éventuellement contenir en outre au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les tensioactifs cationiques, les protéines, les hydrolysats de protéines, les céramides, les pseudocéramides, les acides gras à chaînes linéaires ou ramifiées en C₁₂-C₄₀ tels que l'acide méthyl-18 eicosanoique, les hydroxyacides, les vitamines telles que notamment les vitamines E, C, B, les provitamines tels que le panthénol, les silicones, les huiles animales, minérales ou de synthèse, les polymères anioniques, cationiques, non-ioniques amphotères ou zwitteroniques différents des polymères semi-cristallins selon l'invention, les filtres UV, les parfums, les colorants, les épaississants naturels ou synthétiques, les alcools gras en C₁₂-C₃₀, les agents nacrants, les conservateurs, les agents de stabilisation de pH, les agents antimicrobiens, les agents anti-pelliculaires ou anti-séborrhéiques, les agents anti-oxydants ou réducteurs, les agents acides ou alcalins et leurs mélanges et tout autre additif classiquement utilisé dans le domaine cosmétique qui n'affecte pas les propriétés des composition selon l'invention. De préférence, les compositions sont essentiellement exemptes de silicone, elles comprennent de préférence moins de 1% en poids et plus particulièrement moins de 0,5% et mieux 0% en poids de silicone par rapport au poids total de la composition.

Les compositions de lavage selon l'invention peuvent bien entendu contenir en outre tous les adjuvants usuels rencontrés dans le domaine des shampooings, comme par exemple des parfums, des agents conservateurs, des sequestrants, des adoucissants, des colorants, des agents hydratants, des agents anti-pelliculaires ou anti-séborrhéiques, et autres.

Bien entendu, l'homme de l'art veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Ces compositions peuvent se présenter sous la forme de liquides, éventuellement épaissis, de crèmes ou de gel et elles conviennent principalement au lavage des cheveux. Ces compositions sont de préférence moussantes.

Les compositions peuvent être conditionnées sous diverses formes notamment dans des flacons, des flacons pompe ou dans des récipients aérosols afin d'assurer une application de la composition sous forme vaporisée ou sous forme de mousse.

La présente invention concerne également un procédé de traitement cosmétique des matières kératiniques qui consiste à appliquer une quantité efficace d'une composition telle que décrite ci-dessus, sur les matières kératiniques, à effectuer un rinçage après un éventuel temps de pose.

De préférence, la composition de l'invention se présente sous la forme d'une émulsion huile-dans-eau.

Les compositions conformes à l'invention peuvent être utilisées de préférence en tant que shampoings notamment pour cheveux.

Lorsque les composition conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Les exemples suivants illustrent la présente invention.

### EXEMPLES

### I) Exemples de fabrication de polymères semi-cristallins

### Exemple 1 : Homopolymère de point de fusion de 58°C

Dans un réacteur d'1l muni d'une agitation centrale avec ancre, d'un réfrigérant et d'un thermomètre, on introduit 120g de Parléam que l'on chauffe de la température ambiante à 80°C en 45 min. A 80°C, on introduit en 2h le mélange C₁ suivant :
40g de cyclohexane + 4g de Trigonox 141 [ 2,5 bis (2-éthyl hexanoyl peroxy) - 2,5 - diméthyl hexane].
30 min après le début de la coulée du mélange C₁, on introduit en 1h30 le mélange C₂ constitué de :
200 g d'acrylate de Stéaryle + 400 g de cyclohexane.

A la fin des deux coulées, on laisse agir 3h supplémentaires à 80°C puis on distille à pression atmosphérique la totalité du cyclohexane présent dans le milieu réactionnel.

On obtient alors le polymère à 60 % en poids en matière active dans le Parléam.

Sa masse moléculaire moyenne en poids est de l'ordre de 17 000-27 000 et sa température de fusion T_{f} de 58°C environ, mesurée par D.S.C..

### Exemple 2 : Copolymère de point de fusion de 58°C

On applique le même mode opératoire que dans l'exemple 1, sauf que l'on utilise de l'acrylate de béhényle en lieu et place de l'acrylate de stéaryle. Le polymère obtenu est à 60% en poids en matière active dans le Parléam®. Sa masse moléculaire moyenne en poids est de 23 500-33 500 et sa T_{f} de 58°C environ.

### Exemple 3 : Copolymère de point de fusion de 48°C

On applique le même mode opératoire que dans l'exemple 1, sauf que l'on utilise un mélange de 10 g de N-Vinyl Pyrrolidone et de 190 g d'acrylate de stéaryle.

Le polymère obtenu est à 60% en poids en matière active dans le Parléam, sa masse moléculaire moyenne en poids est de 43 000-53 000 et sa T_{f} de 48°C environ.

### Exemples de formulation :

### Exemple 1

On a préparé des mélanges de polymères semi-cristallins avec les huiles indiqués dans le tableau ci-dessous dans les rapports indiqués ci-dessous :

| Huile | Polymère semi-cristallin | Rapport huile/polymère | Viscosité |
|---|---|---|---|
| Huile d'avocat | POLY C10-30 ALKYL ACRYLATE ⁽²⁾, | 96/4 | 9000 cps |
| Isononanoate d'isononyle | COPOLYMERE ALKYL(C10-30) ACRYLATE/ ACIDE METHACRYLIQUE | 85/15 | 5000 cps |
| Polydécène hydrogéné ⁽¹⁾ | POLY C10-30 ALKYL ACRYLATE ⁽²⁾ | 95/5 | 13700 cps |

| | | | |
|---|---|---|---|
| ⁽¹⁾ vendu sous la dénomination CERAFLOW E par la société SHAMROCK | | | |
| ⁽²⁾ Intelimer IPA13-1 vendu par la société LANDEC | | | |

### Exemple 2

Le shampoing suivant a été préparé. Les proportions sont indiquées en % en poids.

| Composition | Ex 2 |
|---|---|
| Lauryléthersulfate de sodium à 70 % | 14 g (MA) |
| Cocobétaïne à 30 % | 2,8 g (MA) |
| Cocamide MIPA⁽¹⁾ | 1,5 g(MA) |
| Carbomer⁽²⁾ | 0,2 g |
| Polydécène hydrogéné (Ceraflow E- ) | 2,83 g |
| Polymère semicristallin : Intelimer IPA13-1 vendu par la société LANDEC | 0,17 g |
| Hydroxystéaryl céthyl éther et alcool cétylique | 2,5 g |
| Agent de pH qs | PH 7 |
| Conservateur qs | |
| Eau qsp | 100g |

| | |
|---|---|
| MA : Matière Active ⁽¹⁾ vendu sous la marque commerciale Empilan CIS par la société HUNSTSMAN | |
| ⁽²⁾ vendu sous la marque commerciale Carbopol 980 par la société Noveon MA : Matière Active | |

Le polydécène est mélangé au préalable avec le polymère semi-cristallin. Le polydécène pré-épaissi est ensuite introduit dans les tensioactifs.

Cette composition appliquée sur cheveux naturels leur confère après environ 3 minutes de temps de pose et un rinçage, un excellent effet conditionneur.

Des résultats similaires sont obtenus avec les polymères des exemples de fabrication de polymère 1 à 3.

## Revendications

1. Composition détergente, comprenant dans un milieu cosmétiquement acceptable au moins un polymère semi-cristallin ayant un point de fusion supérieur ou égal à 30 °C , au moins une huile et au moins un tensioactif détergent anionique et/ou non ionique, la composition se présentant sous la forme d'une émulsion huile-dans-eau.

2. Composition selon la revendication 1, **caractérisée par le fait que** le polymère semi-cristallin a un point de fusion allant de 30 °C à 80 °C, de préférence allant de 30 °C à 70 °C.

3. Composition selon l'une des revendications précédentes, dans laquelle le polymère semi-cristallin présente une masse moléculaire moyenne en poids supérieur ou égale à 1 000.

4. Composition selon l'une des revendications précédentes, **caractérisée en ce que** le polymère semi-cristallin a une masse moléculaire moyenne en nombre allant de 5 000 à 1 000 000 et mieux de 10 000 à 500 000.

5. Composition selon l'une des revendications précédentes, dans laquelle le polymère semi-cristallin comporte i) un squelette polymérique et ii) au moins une chaîne latérale cristallisable et/ou une séquence organique cristallisable faisant partie du squelette dudit polymère semi-cristallin.

6. Composition selon l'une des revendications précédentes, **caractérisée en ce que** le polymère semi-cristallin est choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

7. Composition selon l'une des revendications précédentes, **caractérisée en ce que** la séquence cristallisable est de nature différente de la séquence amorphe.

8. Composition selon l'une quelconque des revendications précédentes, **caractérisée en ce que** le polymère semi-cristallin a une chaîne organique cristallisable et/ou une séquence cristallisable représentant au moins 30 % et de préférence 40% du poids total du polymère.

9. Composition selon l'une des revendications précédentes, **caractérisée en ce que** le polymère semi-cristallin est choisi parmi :
- les copolymères séquencés de polyoléfines à cristallisation contrôlée,
- les polycondensats de type polyesters,
- les homo- ou co-polymères portant au moins une chaîne latérale cristallisable,
- leurs mélanges.

10. Composition selon l'une des revendications précédentes, caractérisée en ce le polymère semi-cristallin est choisi parmi les homopolymères et copolymères comportant de 50 à 100 % en poids de motifs résultant de la polymérisation de un ou plusieurs monomères porteurs de chaîne(s) latérale(s) hydrophobe(s) cristallisable(s).

11. Composition selon l'une des revendications précédentes, **caractérisée en ce que** le polymère semi-cristallin est choisi parmi les homopolymères et copolymères séquences résultant de la polymérisation d'au moins un monomère à séquence(s) amorphe(s) ou chaîne(s) latérale(s) cristallisable(s) par motif de répétition, leur mélanges de formule (I) : 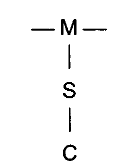 avec M représentant un atome du squelette polymérique, S représentant un espaceur, C représentant un groupe cristallisable et leurs mélanges, avec « S-C » représentant une chaîne alkyle à au moins 11 atomes de carbone, éventuellement fluorée ou perfluorée.

12. Composition selon l'une des revendications précédentes, **caractérisée en ce que** le polymère semi-cristallin est choisi parmi les polymères résultant de la polymérisation d'au moins un monomère choisi parmi l'acide acrylique, méthacrylique, crotonique, itaconique, maléique, l'anhydride maléique et leurs mélanges.

13. Composition selon l'une des revendications précédentes, **caractérisée en ce que** le polymère semi-cristallin est choisi parmi les homopolymères et copolymères résultant de la polymérisation d'au moins un monomère à séquence cristallisable choisis parmi les (méth)acrylates d'alkyle saturés en C₁₄-C₂₄, les (méth)acrylates de perfluoroalkyle en C₁₁-C₁₅, les N-alkyl (méth)acrylamides en C₁₄ à C₂₄ avec ou sans atome(s) de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle comportant de 12 à 24 atomes de carbone, leurs mélanges.

14. Composition selon l'une quelconque des revendications précédentes, **caractérisée en ce que** le polymère semi-cristallin est choisi parmi les copolymères résultant de la polymérisation d'au moins un monomère à chaîne cristallisable choisi parmi les (méth)acrylates d'alkyle saturés en C₁₄ à C₂₄, les (méth)acrylates de perfluoroalkyle en C₁₁ à C₁₅, les N-alkyl (méth)acrylamides en C₁₄ à C₂₄ avec ou sans atome(s) de fluor, les esters vinyliques à chaînes alkyle ou perfluoroalkyle en C₁₄ à C₂₄, les éthers vinyliques à chaîne alkyle ou perfluoroalkyle en C₁₄ à C₂₄, les alpha-oléfines en C₁₄ à C₂₄, les para-alkyl styrènes avec un groupe alkyle comportant de 12 à 24 atomes de carbone, avec au moins un ester ou amide d'acide monocarboxylique en C₁ à C₁₀ éventuellement fluoré.

15. Composition selon l'une quelconque des revendications précédentes, **caractérisée par le fait que** le polymère semi-cristallin est choisi parmi les homopolymères d'alkyl(méth)acrylate ou d'alkyl(méth)acrylamide avec un groupe alkyle en C₁₄ à C₂₄, les copolymères de ces monomères avec un monomère hydrophile.

16. Composition selon l'une quelconque des revendications précédentes, caractérisée en par le fait que les polymères semi-cristallins sont des copolymères d'alkyl(méth)acrylate ou d'alkyl (méth)acrylamide avec un groupe alkyle en C₁₄ à C₂₄ avec un monomère hydrophile comme la N-vinylpyrolidone ou l'hydroxyéthyl (méth)acrylate, et leurs mélanges.

17. Composition selon l'une quelconque des revendications précédentes, **caractérisée en ce que** le polymère semi-cristallin est issu d'un monomère à chaîne cristallisable choisi parmi les (méth)acrylates d'alkyle saturés en C₁₄ à C₂₄.

18. Composition selon la revendication 17, **caractérisée en ce que** le polymère semi-cristallin est choisi parmi les poly(acrylate de stéaryle) ou les poly(acrylate de béhényle).

19. Composition selon l'une quelconque des revendications précédentes, **caractérisée en ce que** le polymère semi-cristallin est présent en une quantité comprise entre 0,005 et 20 % en poids, de préférence entre 0,05 et 10 % en poids par rapport au poids total de la composition.

20. Composition selon l'une quelconque des revendications précédentes, **caractérisée en ce que** l'huile est choisie parmi les huiles végétales, les huiles minérales, les huiles de synthèse, et les esters d'acides gras.

21. Composition selon la revendication 20, **caractérisée en ce que** l'huile est choisie parmi l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; l'huile de paraffine et l'huile de vaseline ; les polydécènes, le squalane, les poly(alpha-oléfines) comme l'isododécane ou l'isohexadécane, les huiles végétales transestérifiées et les huiles fluorées ; les composés de formule RₐCOOR_{b} dans laquelle Rₐ représente le reste d'un acide gras supérieur comportant de 5 à 29 atomes de carbone et R_{b} représente une chaîne hydrocarbonée contenant de 3 à 30 atomes de carbone.

22. Composition selon la revendication 21, **caractérisée en ce que** l'huile est choisie parmi l'huile d'avocat, l'huile de ricin, l'huile d'olive, l'isohexadécane, le polydécène , le myristate d'isopropyle, l'isononanoate d'isononyle, une paraffine liquide.

23. Composition selon l'une quelconque des revendications précédentes, **caractérisée en ce que** la ou le(s) huile(s) est ou sont présente(s) en une quantité comprise entre 0,01 et 30 % en poids, de préférence entre 0,1 et 15 % en poids par rapport au poids total de la composition.

24. Composition selon l'une quelconque des revendications précédentes, **caractérisée en ce que** l'huile est pré-épaissie par le polymère semi-cristallin.

25. Composition selon l'une quelconque des revendications précédentes, **caractérisée en ce que** le rapport pondéral huile(s)/polymère semi-cristallin est supérieur ou égal à 50/50.

26. Composition selon la revendication 25, **caractérisée en ce que** le rapport pondéral huile(s)/ polymère semi-cristallin est supérieur ou égal à 60/40, de préférence compris entre 60/40 et 99/1.

27. Composition selon l'une quelconque des revendications précédentes, **caractérisée en ce que** l'huile présente une taille primaire moyenne en nombre comprise entre 1 et 100 µm, de préférence comprise entre 5 et 30 µm.

28. Composition selon l'une quelconque des revendications précédentes, **caractérisée en ce que** les tensioactifs anioniques sont choisis parmi les sels de métaux alcalins, les sels d'ammonium, les sels d'amines, les sels d'aminoalcools ou les sels de métaux alcalino-terreux, des composés suivants : les sulfates d'alkyle, les alkyléther-sulfates, les alkylamidoéthersulfates, les alkylarylpolyéthersulfates, les monoglycéride-sulfates ; les alkylsulfonates, les phosphates d'alkyle, les alkylamidesulfonates, les alkylarylsulfonates, les alpha-oléfine-sulfonates, les paraffine-sulfonates ; les sulfosuccinates d'alkyle, les alkyléthersulfosuccinates, les alkylamidesulfosuccinates ; les sulfoacétates d'alkyle ; les acylsarcosinates ; et les acylglutamates, les groupes alkyle ou acyle de tous ces composés comportant de 6 à 24 atomes de carbone et le groupe aryle désignant de préférence un groupe phényle ou benzyle ; les esters d'alkyle en C₆-C₂₄ et d'acides polyglycoside-carboxyliques ; les sulfosuccinamates d'alkyle, les iséthionates d'acyle et les N-acyltaurates, le groupe alkyle ou acyle de tous ces composés comportant de 12 à 20 atomes de carbone.

29. Composition selon la revendication 28, **caractérisée en ce que** les tensioactifs anioniques sont choisis parmi les sels de sulfates d'alkyle, d'alkyléther-sulfates, de préférence à 2 ou 3 moles d'oxyde d'éthylène, et d'alkyléther-carboxylates, les groupes alkyle comportant de 6 à 24 atomes de carbone.

30. Composition selon l'une quelconque des revendications 1 à 29, **caractérisée en ce que** les tensioactifs non ioniques sont choisis parmi les alcools, les alpha-diols, les alkyl(C₁-C₂₀)phénols ou les acides gras polyéthoxylés, polypropoxylés ou polyglycérolés, ayant une chaîne grasse, le nombre de groupements oxyde d'éthylène ou oxyde de propylène pouvant aller notamment de 2 à 50 et le nombre de groupements glycérol pouvant aller de 2 à 30 ; les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines ou les oxydes de N-acyl(C₁₀-C₁₄)aminopropylmorpholine.

31. Composition selon la revendication 30, **caractérisée en ce que** les tensioactifs non ioniques sont choisis parmi les alkyl(C₆-C₂₄)polyglycosides et plus particulièrement les alkyl(C₈-C₁₆)polyglycosides.

32. Composition selon l'une quelconque des revendications précédentes, **caractérisée en ce que** les tensioactifs anioniques et/ou non ioniques sont présents en une quantité comprise entre 3 et 50 % en poids, de préférence entre 4 et 30 % en poids par rapport au poids total de la composition.

33. Composition selon l'une quelconque des revendications précédentes, **caractérisée en ce qu'**elle comprend en outre un ou plusieurs tensioactifs amphotères.

34. Composition selon la revendication 33, **caractérisée en ce que** les tensioactifs amphotères sont choisis parmi les alkyl(C₈-C₂₀)bétaïnes, les alkyl(C₈-C₂₀)amidoalkyl(C₆-C₈)bétaïnes, les alkylamphodiacétates.

35. Composition selon l'une quelconque des revendications 33 et 34, **caractérisée en ce que** les tensioactifs amphotères sont présents en une quantité comprise entre 0,1 et 20 % en poids, de préférence entre 0,5 et 15 % en poids par rapport au poids total de la composition.

36. Composition selon l'une quelconque des revendications précédentes, **caractérisée en ce que** le milieu cosmétiquement acceptable comprend l'eau ou un mélange d'eau et d'un solvant cosmétiquement acceptable.

37. Composition selon la revendication 36, **caractérisée en ce que** le solvant cosmétiquement acceptable est choisi parmi les alcools inférieurs en C₁-C₄, les polyols et leurs mélanges.

38. Composition selon l'une quelconque des revendications précédentes, **caractérisée en ce qu'**elle comprend en outre un ou plusieurs additifs classiques choisis parmi des agents tensioactifs cationiques, des polymères anioniques, cationiques, non-ioniques amphotères ou zwitteroniques, des filtres UV, des parfums, des colorants, des épaississants naturels ou synthétiques, des alcools gras en C₁₂-C₃₀, des agents nacrants, des conservateurs, des agents de stabilisation de pH, des vitamines, des provitamines, des agents antimicrobiens, des agents anti-pélliculaires ou anti-séborrhéiques, les agents anti-oxydants ou réducteurs, et les agents acides ou alcalins et leurs mélanges.

39. Composition selon l'une quelconque des revendications précédentes, **caractérisée en ce que** la composition comprend en outre au moins un polymère cationique.

40. Composition selon la revendication 39, **caractérisée en ce que** les polymères cationiques sont choisis parmi les dérivés d'éther de cellulose quaternaires, les gommes de guar cationiques, les cyclopolymères cationiques, les polymères quaternaires de vinylpyrrolidone et de vinylimidazole, les homopolymères ou copolymères éventuellement réticulés de sels de méthacryloyloxyalkyl(C₁-C₄) trialkyl(C₁-C₄)ammonium et leurs mélanges.

41. Composition selon l'une des revendications 38 à 40, **caractérisée en ce que** le ou les polymères cationiques sont présents à des concentrations allant de 0,01 à 20%, de préférence de 0,05 à 10% et plus particulièrement de 0,1 à 5 % en poids par rapport au poids total de la composition.

42. Utilisation de la composition selon l'une quelconque des revendications précédentes, comme shampoing.

43. Procédé de traitement cosmétique des cheveux consistant à appliquer sur les cheveux, une quantité efficace d'une composition telle que décrite à l'une quelconque des revendications 1 à 41, et à rincer après un éventuel temps de pose.
